# EUROPEAN PATENT SPECIFICATION

(11) **EP 2 139 554 B1**
(45) Date of publication and mention of the grant of the patent: **16.05.2012**
(21) Application number: 08745353.6
(22) Date of filing: 09.04.2008
(51) Int. Cl.: A61N 1/362, A61N 1/39, A61N 1/36

(54) **DISCRIMINATION OF SUPRAVENTRICULAR TACHYCARDIA FROM VENTRICULAR TACHYCARDIA**
UNTERSCHEIDUNG ZWISCHEN SUPRAVENTIKULÄRER TACHYKARDIE UND VENTRIKULÄRER TACHYKARDIE
DISTINCTION ENTRE UNE TACHYCARDIE SUPRAVENTRICULAIRE ET UNE TACHYCARDIE VENTRICULAIRE

(30) Priority: 26.04.2007 US 740565
(43) Date of publication of application: 06.01.2010
(73) Proprietor: MEDTRONIC, INC., Minneapolis, Minnesota 55432 (US)
(72) Inventor: ZHOU, Xiaohong, Woodbury, Minnesota 55129 (US)
(74) Representative: Hughes, Andrea Michelle
(86) International application number: PCT/US2008/059723
(87) International publication number: WO 2008/144125

(56) References cited:
- US-A- 5 243 980
- US-A- 5 411 531
- US-A- 5 978 700
- US-A1- 2002 035 335
- US-B1- 7 139 607
- WILKOFF B L ET AL: "Critical analysis of dual-chamber implantable cardioverter-defibrillator arrhythmia detection : results and technical considerations." CIRCULATION 23 JAN 2001, vol. 103, no. 3, 23 January 2001 (2001-01-23), pages 381-386, XP002488383 ISSN: 1524-4539

## Description

### TECHNICAL FIELD

The invention relates generally to implantable medical devices and, in particular, to apparatus for discriminating supraventricular tachycardia from ventricular tachycardia using cardiac neural stimulation.

### BACKGROUND

Implantable cardioverter defibrillators (ICDs) monitor the intervals between electrical depolarizations (observed as P-waves in the atria and R-waves in the ventricles) of a patient's heart for use in detecting atrial and/or ventricular arrhythmias. Tachycardia and fibrillation, referred to collectively as "tachycardia" herein, are detected when a required number of intervals are less than a predefined tachycardia detection interval. The ICD is typically programmed to deliver a therapy in response to a tachycardia detection, which may be anti-tachycardia pacing (ATP) or a cardioversion or defibrillation shock.

Some patients experience atrial tachycardias which are conducted to the ventricles causing a ventricular tachycardia of atrial origin, generally referred to as supraventricular tachycardia (SVT). A therapy delivered to the ventricles in response to detecting a VT that actually originated in the atria will be ineffective in terminating the tachycardia. As such, discrimination of VT and SVT is important in properly diagnosing and treating a patient's heart rhythm, especially in patients who receive a single chamber ICD capable of sensing ventricular signals and not atrial signals. Discrimination of VT and SVT using only cardiac interval monitoring has limited specificity because the ventricular rate during an SVT can sometimes meet VT detection criteria, i.e. the ventricular rates during SVT and during VT can overlap.

US 7,139,607 and US 5,411,531 disclose methods for discriminating between different types of arrhythmia.

### BRIEF DESCRIPTION OF THE DRAWINGS

Figure 1 is a schematic diagram of an implantable medical device (IMD) coupled to a patient's heart for discriminating SVT and VT according to one embodiment of the invention.
Figure 2 is a functional block diagram of the IMD shown in Figure 1.
Figure 3 is a flow chart of a method for discriminating between SVT and VT.
Figure 4 is a flow chart of an alternative method for discriminating between SVT and VT for use in an IMD capable of sensing ventricular signals but not atrial signals.
Figure 5 is a timing diagram illustrating one method for delivering cardiac neural stimulation pulses.
Figure 6 is a flow chart of one method for verifying the effectiveness of cardiac neural stimulation.

### DETAILED DESCRIPTION

In the following description, references are made to illustrative embodiments for carrying out the invention. It is understood that other embodiments may be utilized without departing from the scope of the invention. For purposes of clarity, the same reference numbers are used in the drawings to identify similar elements. As used herein, the term "module" refers to an application specific integrated circuit (ASIC), an electronic circuit, a processor (shared, dedicated, or group) and memory that execute one or more software or firmware programs, a combinational logic circuit, or other suitable components that provide the described functionality.

The invention is defined in claim 1.

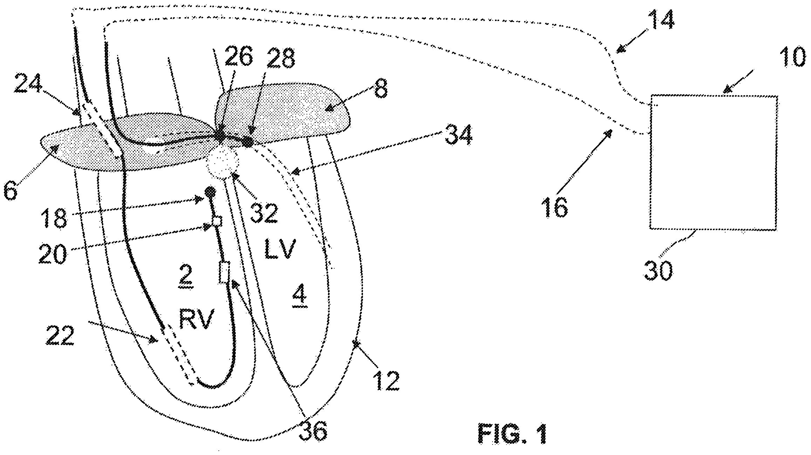

Figure 1 is a schematic diagram of an implantable medical device (IMD) coupled to a patient's heart for discriminating SVT and VT according to one embodiment of the invention. IMD 10 is configured to sense cardiac signals for detecting and discriminating heart rhythms and to deliver electrical stimulation pulses according to programmable operating parameters. Electrical stimulation pulses are delivered for stimulating cardiac neural tissue for discriminating SVT and VT as will be described herein. Electrical stimulation pulses may further be delivered by IMD 10 for pacing heart 12. Such pacing may include bradycardia pacing, cardiac resynchronization therapy, anti-tachycardia pacing or other pacing therapies. IMD 10 may additionally be capable of delivering high energy shock pulses for cardioversion/defibrillation therapy delivered in response to tachycardia detections.

While Figure 1 shows two leads, only one lead having an electrode positioned near the AV node in the base of the right ventricle is needed for achieving both ventricular pacing/sensing and neural stimulation. IMD 10 is coupled to one or more cardiac leads 14 and 16 for use in sensing cardiac signals and delivering stimulation pulses. In the embodiment shown, IMD 10 is coupled to two transvenous leads including a right ventricular (RV) lead 14 and a (CS) coronary sinus lead 16. RV lead 14 includes a distal tip electrode 18 deployed in the basal region of the right ventricle 2 in operative relation to the AV node 32. Ring electrode 20 is spaced proximally from tip electrode 18 for use in bipolar sensing and pacing in the right ventricle. According to one embodiment of the invention, tip electrode 18 is used in conjunction with IMD housing 30 (for unipolar sense/stimulation) or ring electrode 20 (for bipolar sense/stimulation) for sensing ventricular signals for detecting a ventricular rhythm, for delivering cardiac pacing pulses in the right ventricle, and for delivering neural stimulation pulses in the right ventricle for discriminating SVT and VT. RV lead 14 may further include coil electrodes 22 and 24 for use in delivering high-energy shock pulses for cardioversion and defibrillation therapies. Other embodiments may include additional electrodes adapted for sensing and stimulating the right atrium 6, either on a separate right atrial lead or included along RV lead 14. Such electrodes may be positioned relative to the SA node and or AV node for neural stimulation of heart 12.

RV lead 14 further includes a physiological sensor 36 used for sensing signals other than cardiac electrical signals, such as mechanical signals, e.g. pressure, flow, myocardial acceleration, etc., or blood chemistry signals, e.g. temperature, oxygen saturation, pH etc. In one embodiment, sensor 36 is embodied as a pressure sensor for use in verifying effective cardiac neural stimulation.

Coronary sinus lead 16 is deployed in a cardiac vein 34 via the coronary sinus for positioning electrodes 26 and 28 in operative relation to the left chambers of heart 12. In particular, in one embodiment electrodes 26 and 28 are positioned near the AV node 32 to allow stimulation of the cardiac neural tissue for discrimination of SVT and VT. Electrodes 26 and 28 may also be used for sensing cardiac signals and for delivering cardiac pacing pulses in the left ventricle 4. It is recognized that coronary sinus lead 16 may carry additional electrodes such as a coil electrode for use in delivering high energy shock pulses, additional ring electrodes, and/or a tip electrode for cardiac sensing and pacing in the left atrium 8.

The particular lead configuration used may vary between various embodiments of the invention and may include one or more leads, each carrying one or more electrodes. Furthermore, embodiments of the invention are not limited for use with transvenous leads as shown in Figure 1. For example, other embodiments may include the use of epicardial electrodes positioned in operative relation to the fatty pad near the sinoatrial (SA) node and/or the fatty pad near the atrioventricular (AV) node. Subcutaneous electrodes incorporated on the housing 30 of IMD 10 and/or positioned on subcutaneous leads extending from IMD 10 for use in sensing cardiac signals and delivering electrical stimulation pulses for delivering cardiac pacing and shock therapies. Numerous alternative electrode configurations may be appropriate for cardiac neural stimulation, including endocardial or epicardial electrodes deployed near or to the SA nodal and/or AV nodal fatty pads or electrodes positioned along the vagus nerve branches. Cardiac neural stimulation, also referred to herein simply as "neural stimulation", refers to stimulation of neural tissue innervating the myocardium, directly or indirectly, including stimulation of the vagus nerve or its branches, the SA nodal fatty pad, the AV nodal fatty pad and along the great vein.

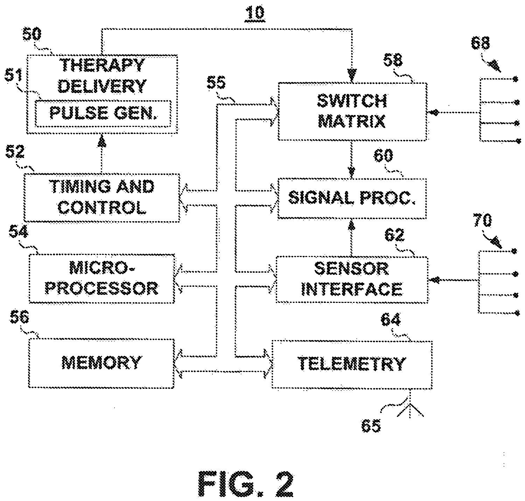

Figure 2 is a functional block diagram of IMD 10 shown in Figure 1. IMD 10 generally includes timing and control circuitry 52 and an operating system that may employ microprocessor 54 or a digital state machine for timing sensing and therapy delivery functions and controlling other device functions in accordance with a programmed operating mode. Microprocessor 54 and associated memory 56 are coupled to the various components of IMD 10 via a data/address bus 55. IMD 10 includes therapy delivery unit 50 for delivering a therapy, such as an electrical stimulation or drug therapy, under the control of timing and control 52. Therapy delivery module 50 includes pulse generating circuitry 51 for generating electrical stimulation pulses under the control of timing and control circuitry 52. As will be described herein, pulse generating circuitry 51 generates stimulation pulses for stimulating neural tissue during tachycardia discrimination algorithms.

For delivering electrical stimulation pulses, pulse generating circuitry 51 may be coupled to two or more electrodes 68 via a switch matrix 58. Switch matrix 58 is used for selecting which electrodes and corresponding polarities are used for delivering electrical stimulation pulses. Electrodes 68 may include lead-based electrodes, leadless electrodes incorporated on IMD 10, and/or the IMD housing configured for use as a can or case electrode. Therapy delivery 50 may further include high voltage circuitry for generating high voltage cardioversion/defibrillation shocks. Aspects of the present invention may be embodied in an implantable cardioverter defibrillator including high voltage circuitry as generally disclosed in U.S. Pat. No. 6,731,978 to Olson et al.

Electrodes 68 may also be used for sensing electrical signals within the body, such as cardiac signals. Cardiac electrical signals are sensed using any of electrodes 68 for detecting the heart rhythm and determining when an electrical stimulation therapy is needed and in controlling the timing of stimulation pulses. As will be described herein, cardiac electrical signals are sensed following delivery of neural stimulation for verifying the effectiveness of the neural tissue stimulation and for discriminating between SVT and VT.

Electrodes used for sensing and electrodes used for stimulation may be selected via switch matrix 58. When used for sensing, electrodes 68 are coupled to signal processing circuitry 60 via switch matrix 58. Signal processor 60 includes sense amplifiers and may include other signal conditioning circuitry and an analog to digital converter. Electrical signals may then be used by microprocessor 54 for detecting physiological events, such as detecting and discriminating cardiac arrhythmias.

IMD 10 may further include physiological sensors 70 such as pressure sensors, accelerometers, flow sensors, blood chemistry sensors, activity sensors or other physiological sensors known for use with IMDs. Sensors 70 are coupled to IMD 10 via a sensor interface 62 which provides sensor signals to signal processing circuitry 60. Sensor signals are used by microprocessor 54 for detecting physiological events or conditions. For example, IMD 10 may monitor heart wall motion, blood pressure, blood chemistry, respiration, or patient activity. Monitored signals may be used for sensing the need for delivering a therapy under control of the operating system. One or more sensor signals may also be used in verifying the effectiveness neural stimulation.

The operating system includes associated memory 56 for storing a variety of programmed-in operating mode and parameter values that are used by microprocessor 54. The memory 56 may also be used for storing data compiled from sensed signals and/or relating to device operating history for telemetry out on receipt of a retrieval or interrogation instruction. IMD 10 further includes telemetry circuitry 64 and antenna 65. Programming commands or data are transmitted during uplink or downlink telemetry between IMD telemetry circuitry 64 and external telemetry circuitry included in a programmer or home monitoring unit.

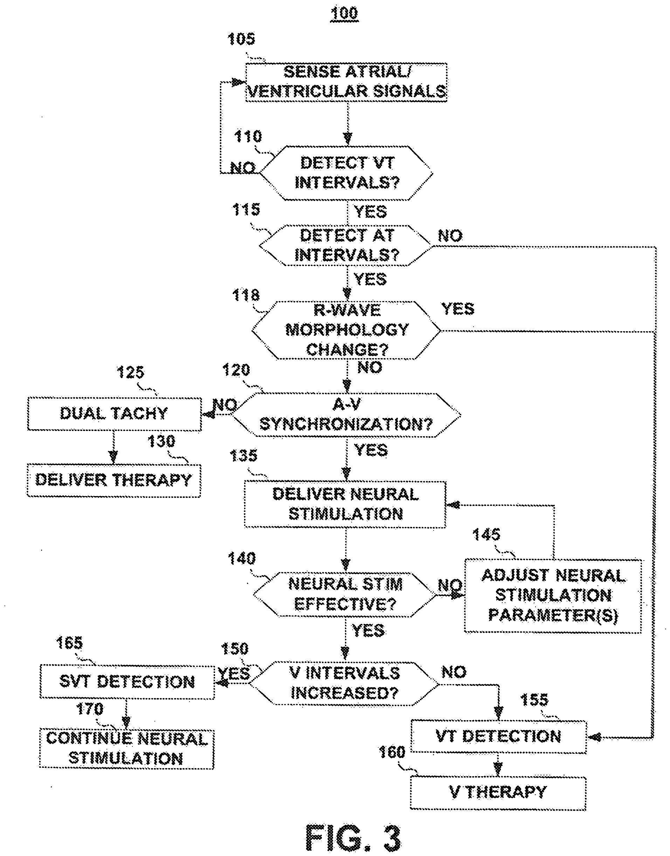

Figure 3 is a flow chart of a method for discriminating between SVT and VT. Flow chart 100 is intended to illustrate the functional operation of the device, and should not be construed as reflective of a specific form of software or hardware necessary to practice the invention. It is believed that the particular form of software will be determined primarily by the particular system architecture employed in the device and by the particular detection and therapy delivery methodologies employed by the device. Providing software to accomplish the present invention in the context of any modern IMD, given the disclosure herein, is within the abilities of one of skill in the art.

Methods described in conjunction with flow charts presented herein may be implemented in a computer-readable medium that includes instructions for causing a programmable processor to carry out the methods described. A "computer-readable medium" includes but is not limited to any volatile or non-volatile media, such as a RAM, ROM, CD-ROM, NVRAM, EEPROM, flash memory, and the like. The instructions may be implemented as one or more software modules, which may be executed by themselves or in combination with other software.

At block 105, the IMD senses cardiac signals to identify intrinsic atrial events (P-waves) and ventricular events (R-waves) for detecting the heart rhythm. The intervals between successive R-waves are compared to a programmed tachycardia detection interval at block 110. If ventricular tachycardia (VT) intervals are not detected, method 100 continues monitoring the sensed intervals. If VT intervals are detected, a determination is made at block 115 whether sensed atrial intervals correspond to atrial tachycardia (AT) intervals. The intervals between successive P-waves are compared to a programmed AT detection interval. The criteria set for determining whether VT intervals are detected and whether AT intervals are detected may require a predetermined number of intervals out of a preceding number of detected RR or PP intervals, respectively, being less than the tachycardia detection interval. Such criteria may correspond to the programmed VT and AT detection criteria or defined separately.

If AT intervals are not detected when VT intervals are present, the IMD detects VT at block 155. The IMD may verify that the VT/VF detection criteria for triggering a therapy delivery are met at block 155 and then proceed in delivering a ventricular therapy to treat the VT/VF according to programmed therapies at block 160.

If AT intervals are detected during the presence of VT intervals, as determined at block 115, the IMD may proceed directly to delivering cardiac neural stimulation at block 135. The presence of the AT intervals may indicate that the VT intervals are due to an SVT rather than an arrhythmia originating in the ventricles. The cardiac neural stimulation will be used to determine if the rhythm is originating in the atria.

At block 118, the R-wave morphology may be examined to determine if the R-wave morphology has changed or corresponds to a VT rhythm. The R-wave morphology may be compared to a template defined for a normal sinus rhythm template. If a change in R-wave morphology is found, a VT detection is made at block 155. Methods for analyzing the R-wave morphology include template matching or comparative analysis of specified characteristics of the R-wave signal. Template matching techniques are generally disclosed in U.S. Pat. No. 6,393,316 (Gillberg et al.), hereby incorporated herein by reference in its entirety.
Prior to delivering cardiac neural stimulation, the IMD may make an additional check at block 120 relating to the synchrony between atrial events and ventricular events. If the ventricular R-waves that are similar to R waves during sinus rhythm are synchronized to atrial P-waves in a 1:1 or other less frequent ratio, the ventricular tachycardia intervals may be the result of a SVT. The test for AV synchrony may include verifying an interval pattern consistent with AV conduction such as an A-V-A-V pattern, A-A-V-A-A-V pattern, etc. The test for AV synchrony may additionally or alternatively include measuring PR intervals, i.e. the intervals between sensed P events and successive R events. If sensed R-events occur within a predetermined interval of a preceding P-event, the R-event is determined to be an atrial conducted event evidencing AV synchrony. A regular pattern of AV synchrony as supported by an AV pattern and/or PR intervals during the occurrence of VT intervals would lead to a detection of AV synchrony at block 120.

If AV synchrony is determined to be present during the detection of VT intervals, neural stimulation is delivered at block 135. If AV synchrony is not present, a dual tachycardia is detected at block 125. Appropriate therapies are delivered in the ventricles for treating the VT, and an atrial therapy may delivered for treating the AT at block 130.

After initiating the neural stimulation at block 135, a verification step is performed at block 140 to verify that the neural stimulation is effective in exciting the neural tissue to cause a parasympathetic response. A parasympathetic response includes any of decrease in heart rate, an increase in AV conduction time, and a decrease in blood pressure. In one embodiment, the verification performed at block 140 includes measuring PR intervals, i.e. the intervals between a sensed P-wave and a sensed R-wave and comparing the PR intervals measured after beginning neural stimulation to PR intervals measured prior to starting neural stimulation, during VT interval detection. Stimulation of the cardiac neural tissue will generally slow AV conduction, resulting in a prolongation of the PR interval. As such a lengthening of the PR interval during cardiac neural stimulation is evidence that the neural stimulation is effectively exciting the neural tissue.

If neural stimulation is not found to be effective, as determined at block 140, the neural stimulation parameters are adjusted at block 145. The number of pulses included in a train of stimulating pulses, the frequency of the pulse train, and/or the amplitude of the stimulation pulses may be increased. When other electrodes are available for stimulating cardiac neural tissue, different electrodes may be selected for stimulating the neural tissue when the neural stimulation is found to be ineffective.

After adjusting the neural stimulation parameter(s), the neural stimulation continues at block 135 and verified as effective at block 140. Once the neural stimulation is found effective, the ventricular intervals, i.e. RR intervals are measured at block 150 during neural stimulation applied using the initial or adjusted stimulation parameters. If the ventricular intervals are increased, i.e. the ventricular rate is decreased, in response to the neural stimulation, the tachycardia is detected as an SVT at block 165. The neural stimulation is sustained at block 170 in response to the SVT detection. The neural stimulation may act to slow a fast atrial rate. The increased ventricular intervals measured at block 150 may be required to increase greater than the VT detection interval in order to detect an SVT. If the ventricular intervals do not increase, and/or remain less than a VT detection interval, a VT detection is made at block 155. A ventricular therapy is delivered at block 160 in response to the VT detection.

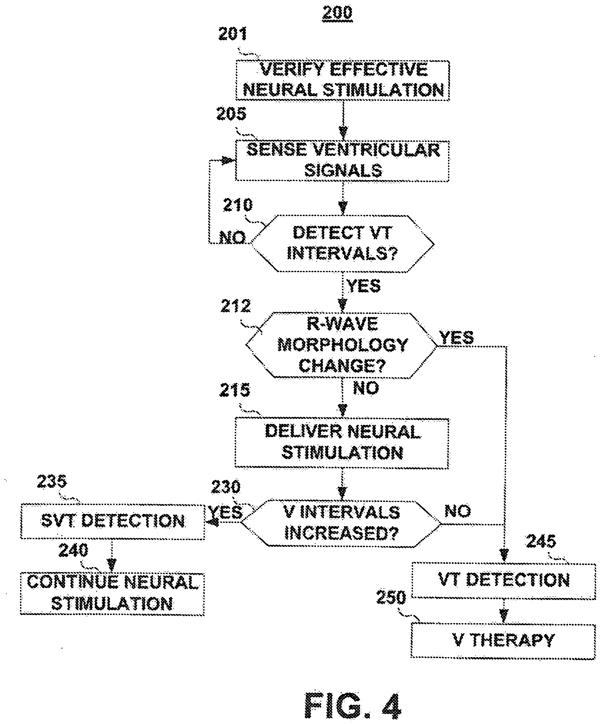

Figure 4 is a flow chart of an alternative method for discriminating between SVT and VT for use in an IMD capable of sensing ventricular signals but not atrial signals. At block 201, neural stimulation is verified as being effective in exciting the cardiac neural tissue to cause a parasympathetic response. In one embodiment, the heart rate is measured during neural stimulation and compared to a heart rate measured just prior to neural stimulation. If the heart rate decreases, cardiac neural stimulation is determined to be effective. In an alternative embodiment, ventricular pressure is measured before and during neural stimulation and a decline in mean blood pressure, mean systolic pressure or another averaged ventricular pressure measurement indicates effective neural stimulation. Neural stimulation parameters, e.g. pulse energy, pulse number and/or pulse frequency, are adjusted until effective neural stimulation is verified at block 201.

At block 205, ventricular signals are sensed for detecting VT intervals as indicated at decision block 210. If VT intervals are detected and the R-wave morphology has changed compared to the R-wave morphology prior to detecting VT intervals, or if the R-wave morphology matches a VT R-wave morphology template or characteristic, VT is detected at block 230. IfVT intervals are detected, and no change in the R-wave morphology is detected at block 212, neural stimulation is delivered at block 215 using the stimulation parameters found to be effective at block 201.

If the ventricular intervals increase in response to the neural stimulation, as determined at decision block 230, an SVT detection is made at block 235. Neural stimulation continues at block 240 for slowing the tachycardia originating in the atria.
If the ventricular intervals do not increase and/or remain shorter than a VT detection interval at block 230, a VT detection is made at block 245. A therapy may be delivered in the ventricle(s) at block 250 for treating the VT. VT therapies may include anti-tachycardia pacing, cardioversion or defibrillation shocks, and/or drug therapies.

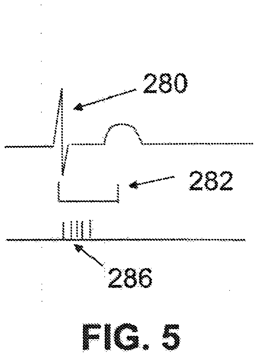

Figure 5 is a timing diagram illustrating one method for delivering cardiac neural stimulation pulses. According to various embodiments of the invention, cardiac neural stimulation pulses 286 are delivered for verifying effective neural stimulation and in response to detecting VT intervals for discriminating between SVT and VT. Neural stimulation pulses 286 are synchronized with ventricular events. In particular, the cardiac neural stimulation pulses 286 are delivered during the ventricular blanking interval 282 applied after a ventricular event 280, which may be a sensed R-wave or a pacing pulse. Blanking interval 282 generally corresponds to a ventricular refractory period following a ventricular sensed or paced event. By delivering the neural stimulation pulses 286 during the ventricular blanking interval 282, the same electrodes used for sensing and/or delivering ventricular pacing pulses may be used for delivering the neural stimulation pulses. In this way, the neural stimulation will not occur during the ventricular vulnerable period thereby avoiding arrhythmogenic effects associated with stimulating during the vulnerable period. For example, when electrodes are positioned in the basal region of the right ventricle for delivering cardiac neural stimulation pulses 282, the same electrodes may also be used for sensing ventricular signals and/or delivering ventricular pacing pulses.

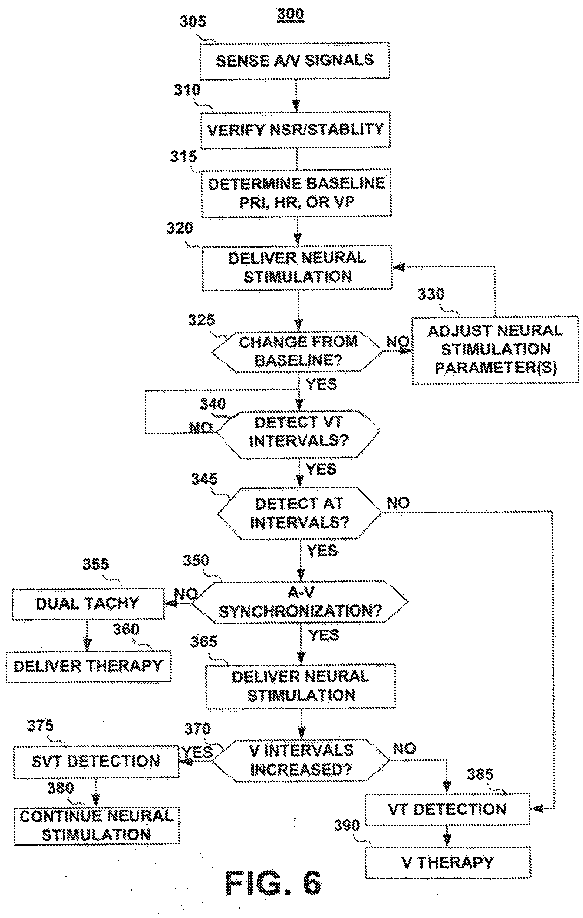

Figure 6 is a flow chart of one method for verifying the effectiveness of cardiac neural stimulation. At block 305, atrial and ventricular signals are sensed for detecting and discriminating heart rhythms. At block 310, a normal sinus rhythm (NSR) is verified based on the atrial and ventricular signals sensed at block 305. Additionally or alternatively, a stable level of physical and/or hemodynamic activity may be verified using a physiological sensor at block 310. For example, a stable level of activity using an activity sensor or other metabolic sensor or a stable blood pressure, heart rate, blood chemistry signal or other physiological signal may be verified. At block 315, a baseline measurement is made of a parameter responsive to parasympathetic stimulation. For example, a baseline PR interval (PRI), heart rate (HR), or ventricular pressure (VP) parameter may be measured. Such measurements are used for verifying the effectiveness of neural stimulation. It is recognized that if the parasympathetic response to the neural stimulation puts the patient at hemodynamic risk, e.g. severe bradycardia, asystole sever fall in blood pressure, the neural stimulation will be adjusted or terminated. For example, in one embodiment, the neural stimulation will be limited to reducing the heart rate to not less than 60 bpm.

At block 320, neural stimulation is initiated. At block 325, the IMD monitors for a change in the baseline parameter measurement indicating the neural stimulation is effective in exciting the neural tissue. One or more criteria may be defmed for establishing effective neural stimulation based, for example, on a decrease in heart rate, increase in PR interval, and/or decrease in ventricular pressure. A threshold may be defined as a function of the baseline measurement(s) for detecting effective neural stimulation. If no change in the baseline measurement is detected, the neural stimulation parameters are adjusted at block 330. The stimulation pulse amplitude, pulse width, pulse number, and/or pulse frequency may be adjusted. When other electrodes are positioned for stimulating neural tissue, a different electrode may be selected at block 330.

Neural stimulation parameters are adjusted at block 330 until effective stimulation is achieved as evidenced by a change from baseline at block 325. The verification of effective neural stimulation performed at blocks 310 through 330 may be performed under clinician supervision upon initial implantation of the IMD or during a patient follow-up visit or automatically on a periodic basis. The IMD monitors ventricular intervals thereafter for detecting VT intervals. Upon detecting VT intervals at block 340, the IMD may check atrial intervals to determine if AT intervals are occurring during the presence of VT intervals at block 345 and optionally whether AV synchronization is present at block 350 as described previously. If AT intervals are not present, a VT detection is made at block 385, and a ventricular therapy may be delivered at block 390 for treating the VT. If AT intervals are present and AV synchronization is not present, dual tachycardia is detected at block 355, and therapies may be delivered in the atria and/or ventricles according to programmed therapy delivery operations at block 360.

If AT intervals are present and synchronized with the VT intervals, neural stimulation is delivered at block 365 using the neural stimulation parameters found to be effective at block 325. If the ventricular intervals are increased in response to the neural stimulation, as determined at block 370, the tachycardia is detected as an SVT at block 375. The neural stimulation continues at block 380 for slowing the fast atrial rate as described previously. If the ventricular intervals do not increase in response to the neural stimulation, a VT detection is made at block 385.

Thus, a device and associated method for discriminating between SVT and VT have been presented in the foregoing description with reference to specific embodiments. It is appreciated that various modifications to the referenced embodiments may be made without departing from the scope of the invention as set forth in the following claims.

## Claims

1. An implantable medical device, comprising:
means (68) for detecting ventricular intervals corresponding to a tachycardia;
means (50) for delivering electrical stimulation pulses to a cardiac neural tissue;
means (52) for verifying the electrical stimulation pulses are effective in exciting the cardiac neural tissue;
means (52) for determining if the ventricular intervals corresponding to the
tachycardia are increased in response to the electrical stimulation pulses; and means for detecting a supraventricular tachycardia in response to the ventricular intervals being increased; **characterized in that** said device is configured to cause said means for delivering electrical stimulation pulses to continue delivering the electrical stimulation pulses in response to the ventricular intervals being increased.

2. An implantable medical device as claimed in claim 1, wherein:
said means for detecting ventricular intervals comprises an electrode (68) for sensing cardiac signals, and a sensing module (70) for receiving the cardiac signals and detecting ventricular intervals corresponding to a tachycardia;
said means for delivering comprises a therapy delivery module (50) for delivering electrical stimulation pulses to cardiac neural tissue; and further comprising a control module (52) coupled to the sensing module and the therapy delivery module and comprising said means for verifying, means for determining, and means for detecting a supraventricular tachycardia;
said control module further configured to control the delivery of the electrical stimulation pulses to the cardiac neural tissue in response to detecting the ventricular intervals corresponding to the tachycardia.

3. The device of claim 1 or 2 wherein verifying the electrical stimulation pulses are effective includes detecting one of an increased atrial-ventricular interval and a decreased heart rate.

4. The device of claim 2 further comprising a blood pressure sensor (70) coupled to the control module for sensing blood pressure signals, wherein verifying the electrical stimulation pulses are effective comprises detecting a decreased blood pressure in response to the electrical stimulation pulses.

5. The device of claim 2 wherein the control module is configured to adjust a parameter controlling the delivery of the electrical stimulation pulses in response to the electrical stimulation pulses not being effective in stimulating the cardiac neural tissue.

6. The device of claim 5 wherein the parameter is one of a stimulation pulse energy, a stimulation pulse frequency, and a stimulation pulse number.

7. The device of claim 5 further comprising a second electrode to deliver cardiac neural stimulation, wherein the control module selects the second electrode in response to the electrical stimulation pulses not being effective in stimulating the cardiac neural tissue.

8. The device of claim 2 wherein the control module detects atrial intervals corresponding to an atrial tachycardia, and wherein the electrical stimulation pulses are delivered in response to detecting the ventricular intervals corresponding to the tachycardia and the atrial intervals corresponding to the atrial tachycardia, and the electrical stimulation pulses are not delivered in response to the atrial intervals not corresponding to the atrial tachycardia.

9. The device of claim 2 wherein the control module detects atrial intervals corresponding to an atrial tachycardia and determines if the ventricular intervals are synchronized to the atrial intervals, and wherein the electrical stimulation pulses are delivered in response to detecting the ventricular intervals being synchronized to the atrial intervals, and the electrical stimulation pulses are not delivered in response to the ventricular intervals not being synchronized to the atrial events.

10. The device of claim 2 wherein the electrode is configured to be positioned along a base of a right ventricle, and wherein the electrical stimulation pulses are delivered using the electrode.

11. The device of claim 10 wherein the electrode is configured to pace the right ventricle.

12. The device of claim 3 wherein the electrical stimulation pulses are delivered during a ventricular blanking interval.

13. A computer-readable medium having computer-executable instructions which, when executed on a device as claimed in any preceding claim, cause said device to perform a method comprising:
sensing cardiac signals;
detecting ventricular intervals from the cardiac signals corresponding to a tachycardia;
delivering electrical stimulation pulses to a cardiac neural tissue;
verifying the electrical stimulation pulses are effective in exciting the cardiac neural tissue;
determining if the ventricular intervals corresponding to the tachycardia are increased in response to the electrical stimulation pulses; and
detecting a supraventricular tachycardia in response to the ventricular intervals being increased; and **characterized by**
continuing delivering the electrical stimulation pulses in response to the ventricular intervals being increased.

## Patentansprüche

1. Implantierbare medizinische Vorrichtung, mit:
Mitteln (68), um ventrikuläre Intervalle, die einer Tachykardie entsprechen, zu detektieren;
Mitteln (50), um elektrische Stimulationsimpulse an ein kardiales Neuralgewebe zu verabreichen;
Mitteln (52), um zu verifizieren, dass die elektrischen Stimulationsimpulse hinsichtlich der Erregung des kardialen Neuralgewebes wirksam sind;
Mitteln (52), um zu bestimmen, ob die ventrikulären Intervalle, die der Tachykardie entsprechen, in Reaktion auf die elektrischen Stimulationsimpulse zunehmen; und
Mitteln, um eine supraventrikuläre Tachykardie in Reaktion darauf, dass die ventrikulären Intervalle zunehmen, zu detektieren; **dadurch gekennzeichnet, dass** die Vorrichtung konfiguriert ist, um zu bewirken, dass die Mittel zum Verabreichen von elektrischen Stimulationsimpulsen die Verabreichung der elektrischen Stimulationsimpulse in Reaktion auf die Zunahme der ventrikulären Intervalle fortsetzen.

2. Implantierbare medizinische Vorrichtung nach Anspruch 1, wobei:
die Mittel zum Detektieren ventrikulärer Intervalle eine Elektrode (68), um kardiale Signale zu erfassen, und ein Erfassungsmodul (70), um die kardialen Signale zu empfangen und um ventrikuläre Intervalle, die einer Tachykardie entsprechen, zu detektieren, enthalten;
wobei die Verabreichungsmittel ein Therapieverabreichungsmodul (50), um elektrische Stimulationsimpulse an kardiales Neuralgewebe zu verabreichen; und ferner ein Steuermodul (52), das mit dem Erfassungsmodul und mit dem Therapieverabreichungsmodul gekoppelt ist und die Mittel zum Verifizieren, die Mittel zum Bestimmen und Mittel zum Detektieren einer supraventrikulären Tachykardie aufweist, enthalten;
wobei das Steuermodul ferner konfiguriert ist, um die Verabreichung der elektrischen Stimulationsimpulse an das kardiale Neuralgewebe in Reaktion auf die Detektion ventrikulären Intervalle, die der Tachykardie entsprechen, zu steuern.

3. Vorrichtung nach Anspruch 1 oder 2, wobei das Verifizieren, dass die elektrischen Stimulationsimpulse wirksam sind, das Detektieren entweder eines vergrößerten atrial-ventrikulären Intervalls oder einer verringerten Herzrate enthält.

4. Vorrichtung nach Anspruch 2, die ferner einen Blutdrucksensor (70) enthält, der mit dem Steuermodul gekoppelt ist, um Blutdrucksignale zu erfassen, wobei das Verifizieren, dass die elektrischen Stimulationsimpulse wirksam sind, das Detektieren eines gesunkenen Blutdrucks in Reaktion auf die elektrischen Stimulationsimpulse enthält.

5. Vorrichtung nach Anspruch 2, wobei das Steuermodul konfiguriert ist, um in Reaktion darauf, dass die elektrischen Impulse hinsichtlich der Stimulation des kardialen Neuralgewebes nicht wirksam sind, einen die Verabreichung der elektrischen Stimulationsimpulse steuernden Parameter einzustellen.

6. Vorrichtung nach Anspruch 5, wobei der Parameter eine Stimulationsimpulsenergie oder eine Stimulationsimpulsfrequenz oder eine Stimulationsimpulsanzahl ist.

7. Vorrichtung nach Anspruch 5, die ferner eine zweite Elektrode aufweist, um eine kardiale Neuralstimulation zu verabreichen, wobei das Steuermodul die zweite Elektrode in Reaktion darauf wählt, dass die elektrischen Stimulationsimpulse hinsichtlich der Stimulation des kardialen Neuralgewebes nicht wirksam sind.

8. Vorrichtung nach Anspruch 2, wobei das Steuermodul atriale Intervalle, die einer atrialen Tachykardie entsprechen, detektiert und wobei die elektrischen Stimulationsimpulse in Reaktion auf die Detektion der ventrikulären Intervalle, die der Tachykardie entsprechen, und der atrialen Intervalle, die der atrialen Tachykardie entsprechen, verabreicht werden und die elektrischen Stimulationsimpulse in Reaktion darauf, dass die atrialen Intervalle nicht der atrialen Tachykardie entsprechen, nicht verabreicht werden.

9. Vorrichtung nach Anspruch 2, wobei das Steuermodul atriale Intervalle, die einer atrialen Tachykardie entsprechen, detektiert und bestimmt, ob die ventrikulären Intervalle mit den atrialen Intervallen synchronisiert sind, und wobei die elektrischen Stimulationsimpulse in Reaktion auf die Detektion, dass die ventrikulären Intervalle mit den atrialen Intervallen synchronisiert sind, verabreicht werden und die elektrischen Stimulationsimpulse in Reaktion darauf, dass die ventrikulären Intervalle nicht mit den atrialen Ereignissen synchronisiert sind, nicht verabreicht werden.

10. Vorrichtung nach Anspruch 2, wobei die Elektrode konfiguriert ist, um längs einer Basis eines rechten Ventrikels positioniert zu werden, und wobei die elektrischen Stimulationsimpulse unter Verwendung der Elektrode verabreicht werden.

11. Vorrichtung nach Anspruch 10, wobei die Elektrode konfiguriert ist, um ein Schrittmachen für den rechten Ventrikel zu bewirken.

12. Vorrichtung nach Anspruch 3, wobei die elektrischen Stimulationsimpulse während eines ventrikulären Austastintervalls verabreicht werden.

13. Computerlesbares Medium, das computerausführbare Befehle besitzt, die dann, wenn sie in einer Vorrichtung nach einem vorhergehenden Anspruch ausgeführt werden, die Vorrichtung veranlassen, ein Verfahren auszuführen, das Folgendes umfasst:
Erfassen kardialer Signale;
Detektieren ventrikulärer Intervalle aus den kardialen Signalen, die einer Tachykardie entsprechen;
Verabreichen elektrischer Stimulationsimpulse an ein kardiales Neuralgewebe;
verifizieren, dass die elektrischen Stimulationsimpulse hinsichtlich der Erregung des kardialen Neuralgewebes wirksam sind;
Bestimmen, ob die ventrikulären Intervalle, die der Tachykardie entsprechen, in Reaktion auf die elektrischen Stimulationsimpulse zunehmen; und
Detektieren einer supraventrikulären Tachykardie in Reaktion darauf, dass die ventrikulären Intervalle zunehmen; und **gekennzeichnet durch**
Fortsetzen des Verabreichens der elektrischen Stimulationsimpulse in Reaktion darauf, dass die ventrikulären Intervalle zunehmen.

## Revendications

1. Dispositif médical implantable, comportant :
des moyens (68) pour détecter des intervalles ventriculaires correspondant à une tachycardie ;
des moyens (50) pour délivrer des impulsions de stimulation électrique à un tissu nerveux cardiaque ;
des moyens (52) pour vérifier que les impulsions de stimulation électrique sont efficaces en excitant le tissu nerveux cardiaque ;
des moyens (52) pour déterminer si les intervalles ventriculaires correspondant à la tachycardie sont augmentés en réponse aux impulsions de stimulation électrique ; et
des moyens pour détecter une tachycardie supraventriculaire en réponse à l'augmentation des intervalles ventriculaires ; **caractérisé en ce que** ledit dispositif est configuré pour amener lesdits moyens de délivrance d'impulsions de stimulation électrique à continuer à délivrer les impulsions de stimulation électrique en réponse à l'augmentation des intervalles ventriculaires.

2. Dispositif médical implantable tel que revendiqué dans la revendication 1, dans lequel :
lesdits moyens de détection d'intervalles ventriculaires comportent une électrode (68) pour détecter des signaux cardiaques, et un module de détection (70) pour recevoir les signaux cardiaques et détecter des intervalles ventriculaires correspondant à une tachycardie ;
lesdits moyens de délivrance comportent un module d'administration de thérapie (50) pour délivrer des impulsions de stimulation électrique à un tissu nerveux cardiaque ; et comportant en outre
un module de commande (52) couplé au module de détection et au module d'administration de thérapie et comportant lesdits moyens de vérification, lesdits moyen de détermination et lesdits moyens de détection d'une tachycardie supraventriculaire ;
ledit module de commande étant également configuré pour commander la délivrance des impulsions de stimulation électrique au tissu nerveux cardiaque en réponse à la détection des intervalles ventriculaires correspondant à la tachycardie.

3. Dispositif selon la revendication 1 ou 2, dans lequel la vérification de l'efficacité des impulsions de stimulation électrique inclut la détection d'un événement parmi un intervalle auriculo-ventriculaire augmenté et une fréquence cardiaque réduite.

4. Dispositif selon la revendication 2, comportant en outre un capteur de pression sanguine (70) couplé au module de commande pour détecter des signaux de pression sanguine, dans lequel la vérification de l'efficacité des impulsions de stimulation électrique comporte la détection d'une baisse de pression sanguine en réponse aux impulsions de stimulation électrique.

5. Dispositif selon la revendication 2, dans lequel le module de commande est configuré pour régler un paramètre commandant la délivrance des impulsions de stimulation électrique en réponse à l'inefficacité des impulsions de stimulation électrique lors de la stimulation du tissu nerveux cardiaque.

6. Dispositif selon la revendication 5, dans lequel le paramètre est un élément parmi une énergie d'impulsion de stimulation, une fréquence d'impulsion de stimulation et un nombre d'impulsions de stimulation.

7. Dispositif selon la revendication 5, comportant également une seconde électrode pour délivrer une stimulation nerveuse cardiaque, dans lequel le module de commande sélectionne la seconde électrode en réponse à l'inefficacité des impulsions de stimulation électrique lors de la stimulation du tissu nerveux cardiaque.

8. Dispositif selon la revendication 2, dans lequel le module de commande détecte des intervalles auriculaires correspondant à une tachycardie auriculaire, et dans lequel les impulsions de stimulation électrique sont délivrées en réponse à la détection des intervalles ventriculaires correspondant à la tachycardie et des intervalles auriculaires correspondant à la tachycardie auriculaire, et les impulsions de stimulation électrique ne sont pas délivrées en réponse aux intervalles auriculaires ne correspondant pas à la tachycardie auriculaire.

9. Dispositif selon la revendication 2, dans lequel le module de commande détecte des intervalles auriculaires correspondant à une tachycardie auriculaire et détermine si les intervalles ventriculaires sont synchronisés sur les intervalles auriculaires, et dans lequel les impulsions de stimulation électrique sont délivrées en réponse à la détection des intervalles ventriculaires synchronisés sur les intervalles auriculaires, et les impulsions de stimulation électrique ne sont pas délivrées en réponse aux intervalles ventriculaires non synchronisés sur les événements auriculaires.

10. Dispositif selon la revendication 2, dans lequel l'électrode est configurée pour être positionnée le long d'une base d'un ventricule droit, et dans lequel les impulsions de stimulation électrique sont délivrées en utilisant l'électrode.

11. Dispositif selon la revendication 10, dans lequel l'électrode est configurée pour stimuler le ventricule droit.

12. Dispositif selon la revendication 3, dans lequel les impulsions de stimulation électrique sont délivrées pendant un intervalle réfractaire ventriculaire.

13. Support lisible par ordinateur ayant des instructions exécutables par un ordinateur qui, lorsqu'elles sont exécutées sur un dispositif tel que revendiqué dans l'une quelconque des revendications précédentes, amènent ledit dispositif à mettre en oeuvre un procédé comportant les étapes consistant à :
détecter des signaux cardiaques ;
détecter des intervalles ventriculaires à partir des signaux cardiaques correspondant à une tachycardie ;
délivrer des impulsions de stimulation électrique à un tissu nerveux cardiaque ;
vérifier que les impulsions de stimulation électrique sont efficaces en excitant le tissu nerveux cardiaque ;
déterminer si les intervalles ventriculaires correspondant à la tachycardie sont augmentés en réponse aux impulsions de stimulation électrique ; et
détecter une tachycardie supraventriculaire en réponse à l'augmentation des intervalles ventriculaires ; et **caractérisé par** l'étape consistant à :
continuer à délivrer les impulsions de stimulation électrique en réponse à l'augmentation des intervalles ventriculaires.
